# EUROPEAN PATENT APPLICATION

(11) **EP 1 568 397 A1**
(43) Date of publication of application: **31.08.2005**
(21) Application number: 04004187.3
(22) Date of filing: 25.02.2004
(51) Int. Cl.: A61N 5/10

(54) **Balloon for brachytherapy and application of the balloon**

(71) Applicant: Acrostak Corp., 8409 Winterthur (CH)
(72) Inventor: Popowski, Youri, 1206 Geneva (CH); Schwager, Michael, 8404 Winterthur (CH)
(74) Representative: Weiss, Wolfgang

(57) **Abstract**

Inflation balloon device and method for irradiation lumpectomy with irradiation consisting of a lumen for filling with fluid, of a lumen for feeding in a wire with a source for ionizing radiation treatment and of a balloon (1) for disposing in an inner cavity (11) of the breast (10) at the site where a tumor was resected, characterized by a balloon (1) which allow an optimal coverage of the inner surface of a cavity (11) created by a removed tumor of the breast (10).

## Description

This invention relates to an inflation balloon device and application of the balloon for irradiation of a lumpectomy cavity with irradiation consisting of a lumen for filling with fluid, of a lumen for feeding in a wire with a source for ionizing radiation treatment and of a balloon for disposing in an inner cavity of the breast at the site where a tumor was resected.

The concept of the accelerated partial-breast irradiation is supported by recent pathologic data and the in-breast failure patterns reported after breast-conservation treatment. Several methods of partial-breast treatment have been established, including techniques utilising multiple catheters implantation, methods of single-fraction intra-operative radiation delivery and partial-breast external beam radiotherapy (Intensity Modulated Radiation Therapy). Multiple-catheter interstitial brachytherapy is presently probably the most widely used technique.

Historically the implantation of multiple catheters was heavily dependant on operator experience with possible variability in target coverage, homogeneity, and overall outcome. Improvements in the technique of catheter placement using various forms of image guidance coupled with 3D - dosimetry now provide the tools for reproducible catheter placement and improved outcome. recently, advancement toward simplification of the brachytherapy processes has been made with the recent clearance of a balloon catheter device. The balloon catheter is placed into the lumpectomy cavity so that the surrounding breast tissue conforms to the balloon surface. Treatment is prescribed and delivered to a circumferential Volume, at 1 cm depth from the balloon Surface, with a central source with high activity(High dose rate).

The early experience seems to show that this device provides reproducible, uniform target coverage with acceptable homogeneity and could be used in selected patients in order to avoid external radiotherapy following surgery. Convenience for the patient is maximized as these approaches deliver all of the radiation during a reduced period of time compared to external radiotherapy. The known intraoperative treatment techniques have raised several questions. AS The placement of the balloon ay be performed during or after the intervention, the availability of the final microscopic margin and axillary nodal status is not always available. Therefore, this information cannot be incorporated into the selection criteria used for the determination of an appropriate treatment approach, which could be a disadvantage of balloon placement during interventions.

The documents US 6,413,204 and US 5,931,774 disclose a round shape balloon that is inserted inside a tumorectomy cavity, in order to perform irradiation of the cavity walls. A radioactive source is placed for an appropriate period of time at the center of the balloon, allowing to deliver the adequate dose, in an homogeneous way to all the periphery of the balloon surface, in order to irradiate homogeneously all the periphery of the scar.

The document US 2003/00929957 A1 discloses a brachytherapy device and method. This invention is directed to a brachytherapy device comprising a substrate comprising at least one radioactive coating layer formed thereon. The radioactive coating layer has a total radioactivity that varies in at least one dimension of the device. Methods of making radioactive coatings, such as electrochemical deposition, electroless deposition and sol-gel are also disclosed Suitable substrates include medical devices and guide-wires, or components thereof. The disclosed methods produce medical devices capable of generating asymmetric, or targeted, radiation fields that correspond to the morphology of a tumor.

Also known is a catheter for intraluminal treatment of a vessel segment with ionizing radiation, see US 6,258,019. This disclosed catheter has an elongated shaft with a proximal and distal END and Has percutaneous transluminal access to the vessel section, an inflatable balloon at the distal end of the shaft, and inflation lumen which runs through the shaft and opens into the balloon, and a source of ionizing radiation which can be positioned in the balloon, and which catheter is filled with gas for applying pressure to the balloon.

The disadvantage of these known devices is the small diameter of the balloons, adapted for intra-vascular treatments (≤ 1 cm in diameter), and, because of only one balloon cylindrical shape type, non homogeneous dose to the complex shape of the most targets.

Therefore the purpose of this invention is achieving a homogeneous dose delivery to a partial-breast target and complete treatment in a short period (5-10 day) with a dose/fractionation scheme similar to that currently used with partial-breast, standard, interstitial brachytherapy. This system could also be used to deliver the boost dose to the lumpectomy cavity after external radiotherapy, in young patients, who are at higher risk for local recurrence, and necessitate higher local doses.

The extent of breast tissue that should be treated after surgical resection of the primary lesion is probably better understood nowadays. When breast-conservation treatment was initiated, whole-breast irradiation was naturally applied as it duplicated the surgical target removed with mastectomy and the radiation techniques used to cover the whole breast were easily applied and widely available leading to high loco-regional control rates. Long-term follow-up of multiple studies investigating standard breast-conserving treatment has proven the role of post-lumpectomy radiation and that whole-breast radiotherapy successfully covers the undefined treatment target. However, it is known standard tangents fields used in whole breast radiotherapy may include excessive volumes of lung and heart leading to some long term complications. In parallel, it is recognized that breast-conserving surgery alone will result in a >20% failure rates in some categories of patients. This does not seem to represent inadequate surgical resection but rather the inadequacy of microscopic margin assessment, unrecognized multifocality of disease and/or unrecognized microscopic disease extent. Finally, based on available pathologic and clinical data, the target requiring treatment with adjuvant radiotherapy after lumpectomy for early stage breast carcinoma in some selected patients seems to be less than whole breast and related to the extent of surgical resection as reflected by the measured microscopic margin.

When considering partial-breast irradiation, the data suggest that a target defined as the lumpectomy cavity plus 1-2 cm margin after resection with negative microscopic margins could be appropriate in selected patients.

The invention proposes a new balloon shape for the treatment of the lumpectomy cavity, that will allow single or multiple intra cavitary implants, to allow an optimal coverage of the inner surface of a cavity of the breast, created by THE removal of a tumor of the breast. The tumor was resected for allowing a balloon implantation, during or several days, weeks after the operation. Moreover several different balloon shapes will allow better adaptation to the resected volumes, pursuant to the invention.

Preferably the inflation balloon device features at least two balloon sections separated by at least one waist, whereas the diameter of the balloon sections are all or partially equal or different. Possible are all thinkable kinds of shapes. All different shaped balloon devices are characterized by balloon sections which are separated by waists.

The envelopes of inflation balloon devices are specially shaped in order to adapt as much as possible to inner cavity of the treated breast. For example a lot of possible shapes are shown in the figures.

Preferably the balloon devices are made of one piece and of one material, for example plastics. As mentioned waists are situated between the balloon sections. No auxiliary means are necessary to stabilize the waists, for example ring elements like in US 6,258,019.

Preferably the shaft of the proximal end of the balloon is made from metal for connection with a loading device for sending the source inside the catheter. This inflation balloon device is applicable to breast brachytherapy characterized by a partial-breast irradiation appropriate for lumpectomy cavity and applicable to a one piece-shaped balloon which is inserted in the lumpectomy cavity for treating with irradiation.

Furthermore a breast brachytherapy is possible by inserting one or more balloons together in the lumpectomy cavity in order to fill the lumpectomy cavity in an appropriate way for adapting the inner shape of the lumpectomy cavity.

Also the inflation balloon device is applicable for treatment of brachytherapy, like cancer of the breast, brain, prostate, uterine, head, neck etc. Furthermore the accurate filling of the lumpectomy cavity can be verified by imaging technique, MRI, ultrasound, CT-scanner, etc.

Some examples of the invention are shown in the figures.

Fig. 1 represents an oval shaped balloon device.

Fig. 2 shows a cylindrical shaped balloon device.

Fig.3 shows a rhombic shaped balloon device.

Fig. 4a and 4b show two kinds of a conical shaped balloon device.

Fig. 5a, 5b and 5c show balloon devices with balloon sections with different diameters.

Fig. 6 shows an application with two balloon catheters.

Fig. 7 shows a balloon device with longitudinal waists.

Fig. 8 shows an example of a balloon device with a curved distal and proximal end.

Fig. 9 shows a solution with a pre-molded shaft.

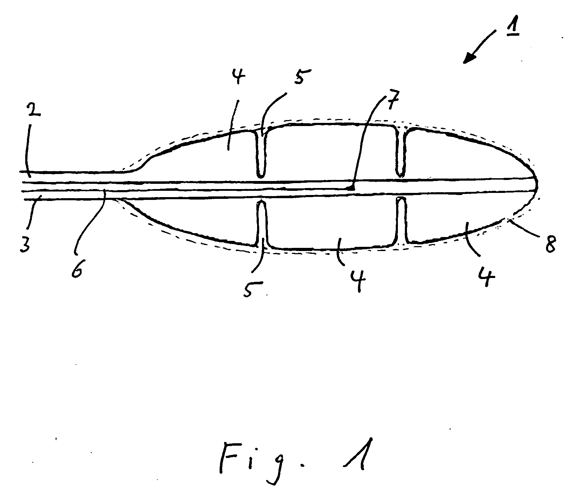

Fig. 1 shows a longitudinal section of a balloon device 1. The envelope 8 of the balloon device 1 is oval shaped. The balloon device 1 has balloon sections 4 separated by waists 5. At the proximal end of the balloon device 1 the lumen 2 for the fluid, the lumen 3 for the wire 6 are arranged. At the top of the wire 6 the source 7 for radiation is situated. The fluid for inflating the balloon device 1 can be liquid or gaseous. All kind of radiation sources are possible, for example radioactive, infrared etc.

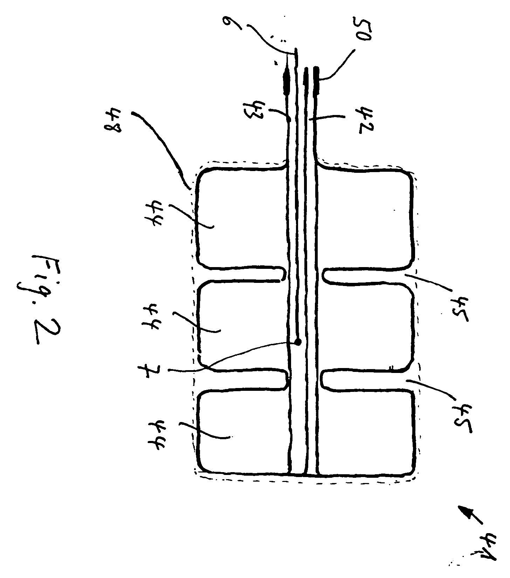

Fig 2 shows a balloon device 41 with a cylindrical envelope 48. Additional to the balloon sections 44, the waists 45, the lumen 42 for the fluid and the lumen 43 for the wire 6 with the radiation source 7 a metallic shaft 50 is arranged. The metallic end is for the connection with a after-loading machine that sends the source inside the balloon device 41.

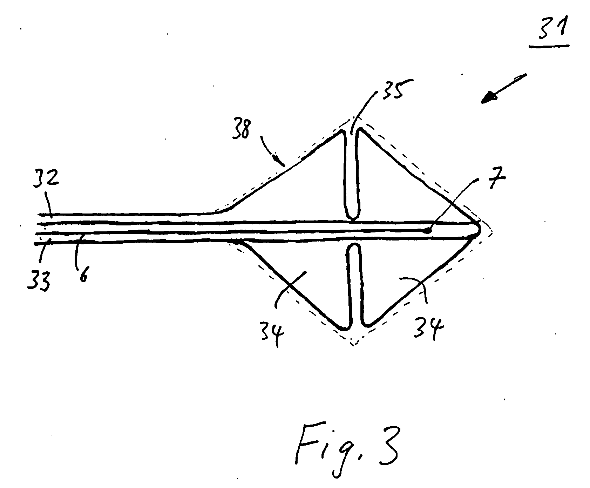

Another balloon device 31 shows Fig. 3. The envelope 38 is like a rhombus. The two balloon sections 34 are separated by the waist 35. The numbers 32 and 33 represent the lumen for the fluid and the lumen for the wire 6 with source 7.

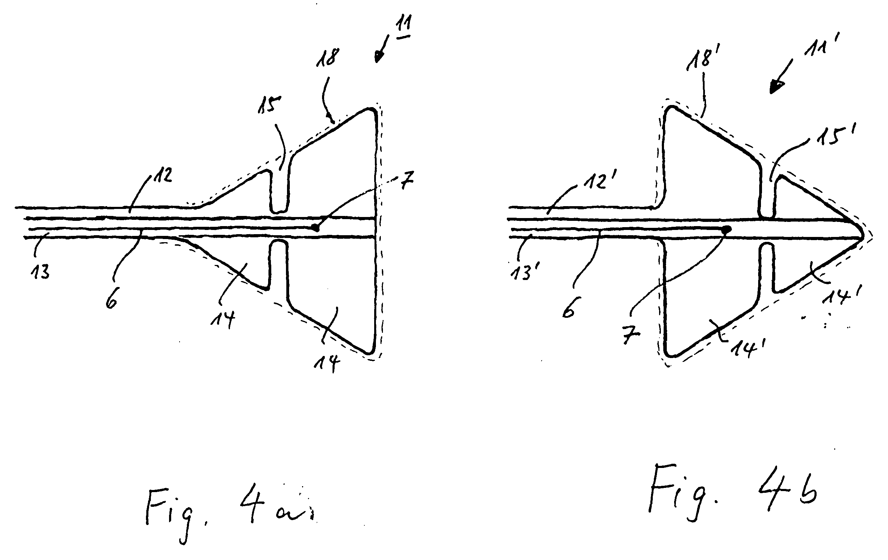

The Fig. 4a and 4b disclose two similar balloon devices 11 and 11' with the balloon sections 14 and 14', the waists 15 and 15', the lumen 12 and 12' for fluid, the lumen 13,13' for the wire 6 with the source 7. In Fig. 4a the part with the smaller diameter of the balloon device 11 is directed to the proximal end. In Fig. 4b the part with the larger diameter is directed to the proximal end.

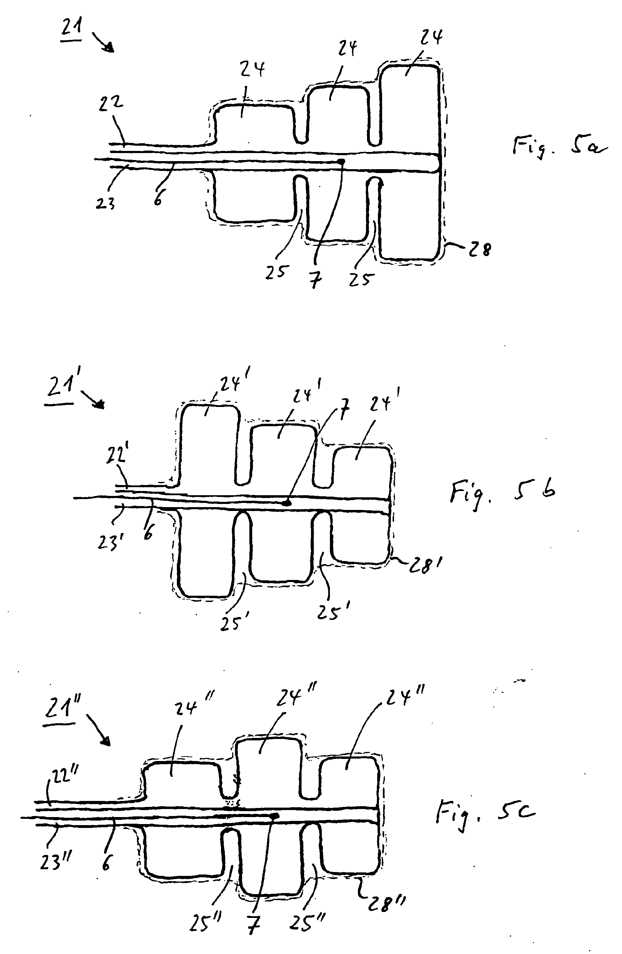

Fig. 5a, 5b and 5c shows examples of balloon devices 21, 21' and 21 ", whereas the envelopes 28, 28' and 28" are stepped. The balloon sections 24, 24' and 24"-separated by the waists 25, 25' and 25" - have different diameters. The exits of the lumen 22, 22' and 22" for the fluid and the lumen 23, 23' and 23"for the wire 6 are can be situated behind the balloon section 24, 24' and 24" with a larger or smaller diameter of the balloon device 21, 21' or 21 ".

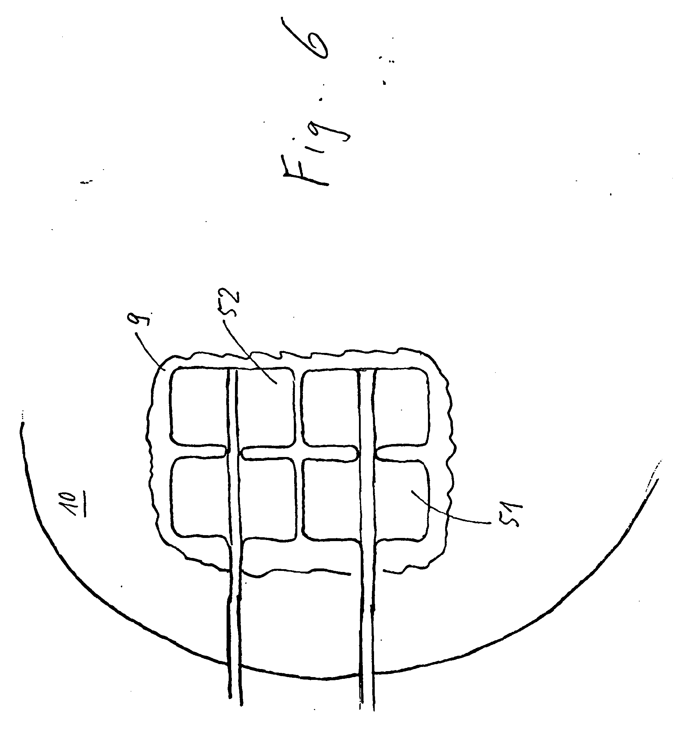

An application of the balloon device shows Fig. 6. Two balloon devices 51 and 52 are inserted in a cavity 9 of a breast 10 for filling the cavity 9 in an optimal way.

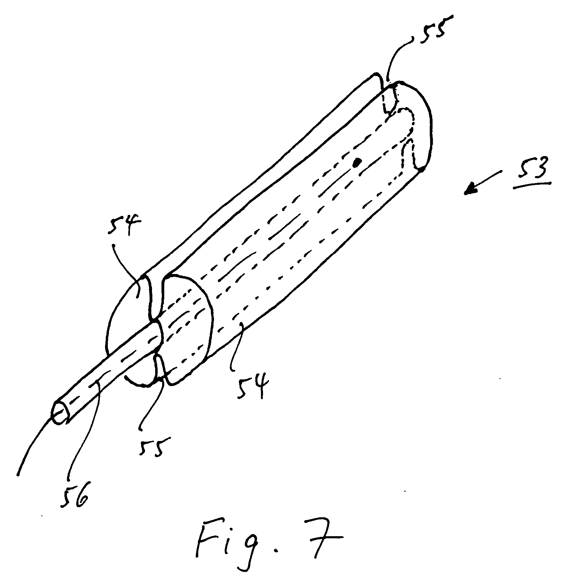

Another possibility shows Fig. 7. In the Fig. 1 till 6 the waists are radial directed. In Fig. 7 the balloon 53 consists of two balloon sections 54 and waists 55 which are directed parallel to the axe 56. The Fig. 7 shows for example a cylindrical shaped balloon 53. Of course the feature of longitudinal directed waist is possible for several other shaped balloons like in the former figures.

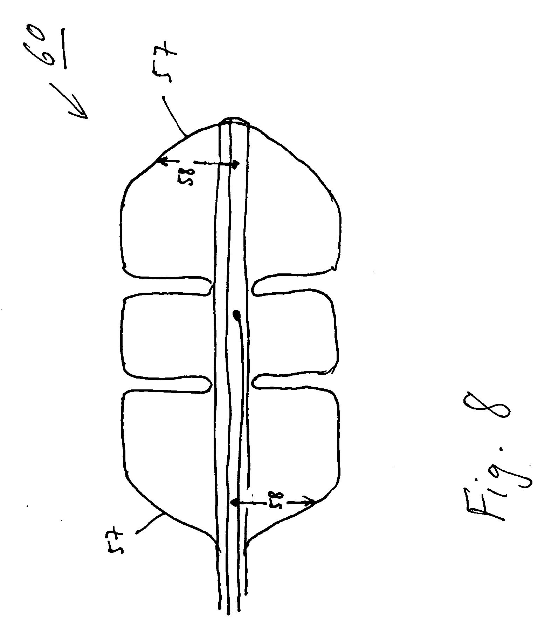

The Fig. 8 shows a further possibility of a balloon device 60. At the proximal and/or the distal end of the balloon device 60 a curved surface 57 is arranged. This curved surface 57 has a decreasing diameter 58. All different shaped balloon devices like in the former figures can have these specially formed ends with decreasing diameter. The advantage of these ends is the better results of dosimetry. With this feature it is possible to adapt the dose of the radiation source to the cavity in an optimal way.

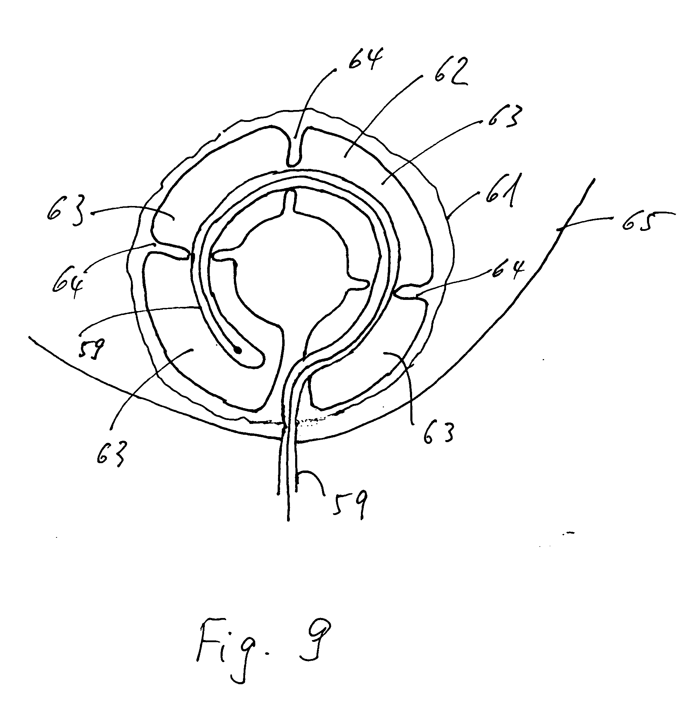

Fig. 9 shows a further example of the invention. Depending of the inner shape of the cavity 61, it is possible to pre-mold the balloon device in a desired shape. In Fig 9 is shown a spherically shaped cavity 61 in a breast 65. The balloon device 62 consists - as former mentioned balloons- of balloon sections 63 with waists 64. In the middle of the balloon device a shaft 59 is arranged. This shaft 59 is pre-molded. In this figure 9 the shaft has a circular shape. But all possible kinds of pre-molded shafts are thinkable, depending of the inner shape of the treated cavity.

The above described features of the invention are merely exemplary and not limited. All thinkable shapes of balloon devices are possible. The main point is to find an appropriate shape for filling a the cavity and for obtaining an optimal radiation treatment.

## Claims

1. Inflation balloon device for irradiation of a lumpectomy cavity (9) with irradiation consisting of a lumen (2, 12, 12', 22, 22', 22", 32, 42) for filling with fluid, of a lumen (3, 13, 13', 23, 23', 23", 33, 43) for feeding in a wire (6) with a source (7) for ionizing radiation treatment and of a balloon (1, 11, 11', 21, 21', 21" ,31, 41) for disposing in an inner cavity (9) of the breast (10) at the site where a tumor was resected, **characterized by** a balloon (1, 11, 11', 21, 21', 21" ,31, 41) which allow an optimal coverage of the inner surface of a cavity (9) created by a removed tumor of the breast (10).

2. Inflation balloon device of claim 1 **characterized by** a balloon (1, 11, 11', 21, 21', 21" ,31, 41) with at least two balloon sections (4, 14, 14', 24, 24', 24", 34, 44) separated by at least one waist (5, 15, 15', 25, 25', 25", 35, 45), whereas the diameter of the balloon sections (4, 14, 14', 24, 24', 24", 34, 44) are all or partially equal or different.

3. Inflation balloon device of claim 1 or 2 **characterized by** a balloon (1, 11, 11', 21, 21', 21" ,31, 41) of which the envelope (8, 18, 18', 28, 28', 28", 38, 48) is specially shaped in order to adapt as much as possible to inner cavity (9) of the treated breast (10).

4. Inflation balloon device of claim 3 **characterized by** a balloon (1) of which the envelope is oval shaped.

5. Inflation balloon device of claim 3 **characterized by** a cylindrical shaped balloon (41).

6. Inflation balloon device of claim 3 or 5, **characterized by** a balloon (41) of which the diameter is superior to 1 cm.

7. Inflation balloon device of claim 3 or 6, **characterized by** a balloon (53) with at least two balloon sections (54) separated by at least one waist (55) whereas the waist (55) is directed parallel to the axe (56) of the balloon device.

8. Inflation balloon device of claim 3 **characterized by** a balloon (31) of which the envelope (38) is rhombic shaped.

9. Inflation balloon device of claim 3 **characterized by** a balloon (11, 11') of which the envelope (18, 18') is conical shaped, whereas the thinner end of the balloon device is directed either to the distal or to the proximal end.

10. Inflation balloon device of claim 3 **characterized by** at least two balloons (21, 21', 21") of which the longitudinal section is stepped, whereas the diameters of the balloons are different or partially equal.

11. Inflation balloon device of claim 9 **characterized by** a balloon (21, 21', 21") whereas the balloon section with the smallest diameter is directed either to the distal or to the proximal end.

12. Inflation balloon device of at least one of the former claims **characterized by** a balloon device that the distal and/or to the proximal end has a surface (57) with a decreasing diameter (58).

13. Inflation balloon device of at least one of the former claims **characterized by** a shaft (59) which is pre-molded.

14. inflation balloon device of claim 13 **characterized by** a pre-molded shaft (59) which is circular or semicircular.

15. Inflation balloon device of at least one of the former claims **characterized by** a balloon (1, 11, 11', 21, 21', 21" ,31, 41) which is made of one piece and one material.

16. Inflation balloon device of at least one of the former claims applicable for a catheter, whereas the shaft (50) of the proximal end of the balloon (1, 11, 11', 21, 21', 21" ,31, 41) is made from metal for connection with a loading device for sending the source inside the catheter.

17. Inflation balloon device of at least one of the former claims applicable to a method for breast brachytherapy **characterized by** a partial-breast irradiation appropriate for lumpectomy cavity (9).

18. inflation balloon device of at least one of the former claims applicable to a method for breast brachytherapy **characterized by** a one piece-shaped balloon which is inserted in the lumpectomy cavity (9) for treating with irradiation.

19. inflation balloon device of at least one of the former claims applicable to a method for breast brachytherapy **characterized by** one or more balloons inserted together in the lumpectomy cavity (9) in order to fill the lumpectomy cavity (9) in an appropriate way for adapting the inner shape of the lumpectomy cavity (9).

20. Inflation balloon device for irradiation lumpectomy with irradiation and method applicable for treatment of brachytherapy, like cancer of the breast, brain, prostate, uterine, head, neck etc.
